# EUROPEAN PATENT SPECIFICATION

(11) **EP 1 401 407 B1**
(45) Date of publication and mention of the grant of the patent: **31.08.2005**
(21) Application number: 02735448.9
(22) Date of filing: 11.06.2002
(51) Int. Cl.: A61K 9/70, A61K 33/30, A61K 33/34, A61K 33/04, A61K 33/26, A61L 15/44, A61P 17/02, A61P 31/00

(54) **POROUS SPONGE-LIKE CELLULOSIC MATERIAL FOR TREATMENT OF INJURIES**
PORÖSES SCHWAMMARTIGES ZELLULOSEMATERIAL ZUR BEHANDLUNG VON VERLETZUNGEN
MATIERE CELLULOSIQUE POREUSE DE TYPE ABSORBANTE DESTINEE AU TRAITEMENT DE PLAIES

(30) Priority: 12.06.2001 FI 20011232; 12.06.2001 US 297269 P
(43) Date of publication of application: 31.03.2004
(73) Proprietor: Vivoxid Oy, 20520 Turku (FI)
(72) Inventor: VILJANTO, Jouko, FIN-20720 Turku (FI); PENTTINEN, Risto, FIN-20720 Turku (FI); LÖNNQVIST, Kurt, FIN-20500 bo (FI)
(74) Representative: Nordin, Leif Göran
(86) International application number: PCT/FI2002/000503
(87) International publication number: WO 2002/100383

(56) References cited:
- EP-A- 0 206 692
- EP-A- 0 238 839
- WO-A-98/44964
- GB-A- 887 844
- GB-A- 1 068 667
- US-A- 5 079 010
- US-A- 6 060 079
- JOUKO VILJANTO ET AL: "Local hyperalimentation of open wounds" BR.J.SURG, vol. 63, no. 6, 1976, pages 427-430, XP002902689
- DATABASE WPI Section Ch, Week 200133 Derwent Publications Ltd., London, GB; Class A96, AN 2001-315129 XP002902690 & RU 2 164 426 C (LASER MED RES CENTRE), 27 March 2001 (2001-03-27)

## Description

The present invention relates to a sponge-like cellulosic material for treatment of injuries and/or burns and the use of said material defined in the preambles of the independent claims presented hereafter.

There exists a need for cheap and easy-to-use material, which can be used in treatment of injuries and/or burns before skin transplantations. Usually patients with injuries such as large third degree burns or with large wounded areas are in the need of skin transplants. However, it is not always possible to carry out the skin transplantation immediately after the injury because of insufficient circulation or heavy contamination of the wound base. If area of the injured tissue is too large the patient may not have enough skin left for autografts, or the patient can be so seriously injured that the operations have to be postponed until the patient has sufficiently recovered. Before the skin transplantation dead and devitalised tissue has to be removed and injured tissues have to be temporarily covered to minimise the fluid, electrolyte and protein losses from the injured tissues, to maintain adequate body temperature and to promote wound base granulation and revascularisation of the damaged tissue. Injuries, such as large open wounds and burns are extremely susceptible to infections if they are not covered.

The coverings that are used today include different artificial skin replacements and traditional temporary coverings, such as wound dressings. Skin replacements, so called artificial skins or living skin equivalents, contain usually tissue-based or tissue-related products, such as collagen. They can also contain living skin cells, such as cultured human fibroblasts, and proteins, such as glycosaminoglycan. The drawback of this type of skin replacements is their price, as they are extremely expensive: costs for a patient with severe 50% burns can add up to 150-200,000 FIM. In addition to costs, certain doubts and health risks are related to their use. It is not yet known if the skin replacements containing proteins may act as a possible source for prion-induced diseases, e.g. Creutzfeldt-Jakob disease. The polymer-based matrices can contain small amounts of monomers or polymerisation catalyst residues that can be detrimental or even toxic to the tissues. These skin replacements do not effectively prepare the wound base for transplantation, either. Human skin originating from cadavers has been used as skin allografts. Cadavers are tested for infectious diseases, such as hepatitis B and C and HIV, but especially in case of HIV, which has relatively long incubation time, fresh infections may pass screening tests unnoticed.

Cellulose viscose sponge has also been used as temporary cover for burned areas (Viljanto, J. et Jääskeläinen, A. "Stimulation of Granulation Tissue Growth in Burns", Annales Chirurgiae et Gynaecologiae Fenniae 62 1973). The results with cellulose viscose sponge were promising, but in order to improve the wound base granulation and revascularisation it was necessary to continually moisten the sponge with a mixture of different amino acids and vitamins (Viljanto, J. et Raekallio, J. "Local Hyperalimentation of Open Wounds", The British Journal of Surgery 6:63, 1976). The continuous moistening was difficult to perform and to control satisfactorily. Further, amino acids are sensitive and their mixture cannot be stored for longer times than for a few days. This made it necessary to prepare fresh amino acid mixture regularly as the continuous moistening of the sponge was required. Quite naturally the complicated procedure, with which the improved results in wound base granulation and revascularisation were obtained, diminished the interest to use cellulose viscose sponge in treatment of injuries and/or burns for the following decades.

### BRIEF DESCRIPTION OF THE INVENTION

The object of the present invention is to provide a cost effective and easy-to-use spongy cellulosic material for treatment of injuries and/or burns.

The object is thus to provide an improved alternative to existing cellulosic sponge-like materials.

Another object of the invention is the use of the novel spongy cellulosic material in treatment of injuries, such as burns or acute or chronic wounds.

In order to achieve the above-mentioned objects the present invention is characterised in what is defined in the characterising parts of the independent claims presented hereafter.

A typical porous sponge-like cellulosic material to be used in treatment of injuries and/or burns comprises zinc, copper, selenium and/or iron bound to and/or absorbed into the material matrix in amounts sufficient to support wound base granulation and revascularisation. The amount of zinc, copper, selenium and iron is at least 0.1 µg/g dry material.

### DETAILED DESCRIPTION OF THE INVENTION

Now it has been surprisingly found out that by incorporating inorganic trace elements that are essential in wound base granulation and revascularisation, similar results can be obtained than when using cellulose viscose sponge moistened with complicated amino acid and vitamin mixtures. The present invention provides a simple and cheap alternative with which improved treatment results of injuries and/or burns before skin transplantation are achieved.

The term "sponge-like cellulosic material" when used in the context of the present invention relates to material, which is made of cellulose and which contains micro- and macropores in contact with each other. The material has sponge-like, i.e. spongy, physical characteristics: it is soft, light, porous, compressible, elastic and it is capable of absorbing liquids.

The term "trace element" when used in the context of the present invention relates to inorganic chemical elements and substances, which are needed in trace amounts to ensure the specific functions of human body, such as development and tissue growth. Such elements are for example iron, copper, zinc, selenium, magnesium and manganese.

According to the invention suitable trace elements, which promote wound base granulation and revascularisation are incorporated to the material matrix of the porous spongy cellulosic material. Trace elements can be incorporated to the material matrix by any suitable method known as such. They can be incorporated to the matrix for example by absorption, adsorption, impregnation or they can be chemically bound to the matrix. The elements can be trapped into the material matrix with different physical or chemical methods. The trace elements can e.g. be deposited into the material matrix in the production process of the sponge-like cellulosic material, the trace elements then forming a part of the material structure. As the material matrix as such is made of cellulose, it is free from all chemical residue problems associated with polymer-based materials.

For example, the sponge-like cellulosic material according to the present invention can be prepared by preparing a cellulosic sponge by a method known as such. The spongy cellulosic material can be washed repetitively with distilled water. The clean spongy cellulosic material thus obtained can be washed with a solution containing desired trace elements. During the washing trace elements will be deposited on the material matrix and amount of deposited elements can be easily calculated from concentrations remaining in the washing liquid. After the deposition of trace elements the sponge-like cellulosic material can be dried, cut into desired shape and packed.

According to one preferred embodiment of the present invention the sponge-like cellulosic material comprises at least zinc, copper, selenium and/or iron. The material can comprise only one of these trace elements, or two or more at the same time. In addition to these elements the material can also comprise other trace elements, if they are deemed to be beneficial for the wound base granulation and revascularisation. The total amount of zinc, copper, selenium and iron is normally at least 0.1 µg/g dry material. In one embodiment of the present invention the total amount of zinc, copper, selenium and iron is typically at least 0.3 µg/g dry material, sometimes at least 0.5 µg/g dry material.

According to other embodiments of the invention the zinc concentration can be 2 - 20 µg/g dry material, preferably 7 - 10 µg/g dry material, the iron concentration 5 - 28 µg/g dry material, preferably 10 - 14 µg/g dry material, the copper concentration 4 - 24 µg/g dry material, preferably 6.0 - 10 µg/g dry material, and/or the selenium concentration 0.5 - 3.0 µg/g dry material, preferably 0.8 - 1.2 µg/g dry material. According to invention the concentrations of zinc, copper, selenium and iron can be selected independently from each other, and it is not necessary to have them all simultaneously in the material matrix.

In one embodiment of the invention the copper concentration is 9.5 -12 µg/g dry material and/or the selenium concentration 1.1 - 1.4 µg/g dry material.

In addition to above-mentioned trace elements cellulose sponges can also be used to deliver calcium and phosphate locally to cells. This will greatly enhance the cellular proliferation and synthesis of cellular and/or extra cellular components. Similar principle has been used for two hundred years in agriculture to enhance cellular growth as fertilizer.

The material according to the invention promotes effective connective tissue regeneration and wound base granulation, and it is easy to use and cheap in price. The material needs only to be moistened with physiological saline solution or the like before placing it on injured tissue. No continuous infusion with complicated amino acid solutions is necessary for obtaining successful wound base granulation and revascularisation. The simplicity of the use and inexpensiveness renders the material according to the present invention suitable to be used also in difficult and inadequate conditions, e.g. in developing countries or field hospitals.

When used in treatment of injuries the material according to invention is cut into a suitable shape and placed on the cleaned injured tissue. The sponge-like cellulosic material can attract transforming growth factor beta 1 (TGF-β1), which is an inhibitor of epithelial cell growth, into the material and to the material/tissue interface. Thus, material can effectively inhibit the growth of keratinocytes at the wound base, and prevent the unwanted keratinisation of the wound base. Similarly, the material according to the present invention can promote disappearance of small regions of skin, if such regions exist at the wound base. Removal of keratinous tissue and inhibition of keratinisation is important, as it will improve the success of the following skin transplantation.

When placed on the wound base the material according to the invention will function as a protein adsorbent and as a good hemostatic. The material will stick tightly to the wound base, and promote the growth of the capillaries also into the material. When the material is removed from the wound base, capillaries that have grown inside the sponge-like material are broken, which will render the wound base suitable for grafting.

According to invention the porous sponge-like cellulosic material comprising trace elements is preferably used in treatment of injuries, such as deep burns and different types of acute or chronic wounds. The use of the invention will prevent the local depletion of the essential trace elements in the injured tissue, and guarantee the availability of e.g. iron(II), copper, zinc, selenium, magnesium and/or manganese, which are needed for optimal wound base granulation. These trace elements are usually provided by intravenous alimentation, but they can reach the injured tissue only if it is well vascularised. For example, in deep third degree burns the injured tissue is practically lifeless: epidermis is totally destroyed and dermis partially or totally destroyed, and vascularisation in the injured tissue is lacking. In such cases local depletion of the essential trace elements occurs, even if a solution containing trace elements were given to the patient intravenously. The result is that the regenerative capacity of the patient is exceeded, resulting in poorly granulating and easily infected surfaces. This depletion of essential trace elements can be avoided when material according to the present invention is used in treatment of such injuries. The present invention therefore fulfils the long felt need for material, which is effective in promoting wound base granulation and revascularisation and at the same time easy to use and cost-effective.

According to one embodiment of the invention the spongy cellulosic material comprising trace elements can be also used for example as first aid in acute wounds, such as traffic or occupational accidents. The possibilities to take care of these injuries in local health care centres or smaller hospitals are often limited, and patients have to be transferred to larger specialised care units. During the patient transportation the raw surface of the injured area is simply covered with the material according to the invention, thus preventing diffuse bleeding, fluid loss and the contamination of the injured tissue.

The material according to invention can be used in treatment of injured tissues, where not only the epidermal but also the dermal part of the skin is partially or totally destroyed. Such injuries are, for example:
- severe burns,
- acute wounds, caused by e.g. occupational or traffic accidents,
- chronic ulcers, e.g. diabetic, varicose and trophic ulcers,
- pressure ulcers, and
- chronic infections, e.g. leprosy.

One aspect of the present invention is therefore the use of porous sponge-like cellulosic material comprising trace elements for the manufacture of a device for treatment of acute wounds, burns, chronic ulcers or chronic infections.

According to one embodiment of invention the spongy cellulosic material comprising trace elements can also be used in treatment of infected injuries. It has been observed that when moistened and placed on the raw surface of the injured tissue, the material according to invention will "attract" mononuclear phagocytes, such as monocytes and macrophages to the interface between the material and the infected tissue. As these phagocytes effectively destroy bacteria, extraneous matter and dead cells causing the infection, the infection can be removed from the wound base by changing the spongy cellulosic material comprising trace elements as often as necessary until the tissue surface is ready for grafting.

The material according the invention can especially be used in treatment of infected tissue cavities, which are otherwise difficult to treat. The orally administered antibiotics do not reach these tissues via blood circulation if the tissues are poorly vasculariscd. The spongy cellulosic material comprising trace elements can simply be moistened with physiological saline or Ringer's solution, put into the cavity and changed as necessary. As described above, the material will support the body's own mechanisms to overcome the infection in the tissue. At the same time, as the use of the material will improve the revascularisation of the tissue, also orally administered antibiotics will reach it.

According to one embodiment of the invention a semi-permeable film is attached on one side of the porous sponge-like cellulosic material comprising trace elements.

Preferably the water vapour can permeate through the film, but it can detain proteins with molecular weight > 5000 in the material. According to preferred embodiment the semi-permeable film will detain proteins bigger or of similar size as albumin and fibrinogen. When the film is attached on the material according to invention, it will be easier to use, and by controlling the properties of the attached film, e.g. pore size and film thickness, the fluid losses through the film can be controlled more accurately. The semi-permeability of the film can also change as a function of temperature. At higher temperatures the permeability of the film is preferably lower than at lower temperatures, as the secretion of fluid from the injured tissue is usually increased at elevated temperatures. Importance of the detention of the moisture in the material is especially pronounced in the cases where patients treated with the material according to invention are kept under heat generating lamps, at elevated temperatures up to 27 - 28 °C.

The porous and spongy cellulosic material comprising trace elements can be easily formed in different shapes and sizes. Devices, such as sheets, pieces, slips, slices and other articles can be easily made for treatment of burns, acute wounds, chronic rn ulcers and chronic infections. According to one embodiment of the invention it is also possible to obtain a ready-to-use product by moistening the sterilised ready-cut cellulose material comprising trace elements with physiological salt solution, and pack it in hermetically sealed packages. The material according the present invention can be formed as sheets that are easy to use in first aid for example on the scene of an occupational or traffic accident.

### DESCRIPTION OF THE DRAWINGS

The invention is further illustrated in the following drawings that show some embodiments of the invention.

Figures 1a - 1c illustrate schematically a piece of spongy cellulosic material comprising trace elements placed on the raw surface of the injured tissue, and

Figures 2a - 2b demonstrates a rapid and quantitative binding of transforming growth factor beta 1 (TGF-β1) into cellulose sponges in vitro.

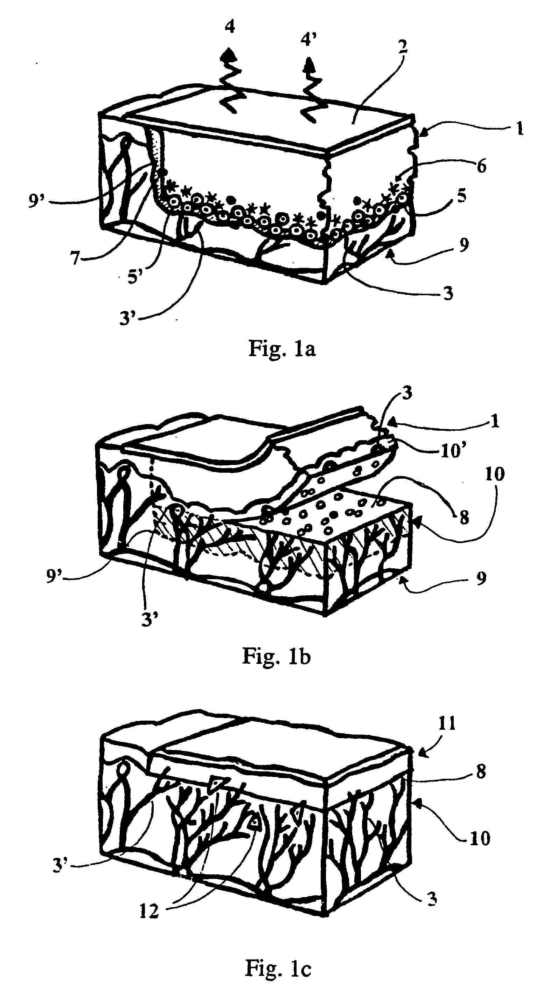

Figure 1a shows a piece 1 of sponge-like cellulosic material according to the present invention comprising trace elements. The piece 1 has been placed on the raw infected surface 9' of the injured tissue 9. On the piece 1 a semi-permeable film 2 has been attached. Film 2 allows evaporation of water vapour from the piece 1. Evaporation of water has been indicated with arrows 4, 4'. Typically about 5 mg H₂O/cm³/h is evaporated. Phagocytes 5, 5' are attracted to the surface region of the infected surface 9' and also into the piece 1, where they destroy bacteria 6, indicated as stars, and debris 7, indicated as black dots. Blood vessels 3, 3' do not reach the surface 9' of the injured tissue 9.

In figure 1b blood vessels 3, 3' have grown partially inside the piece 1 of sponge-like material and the wound base revascularisation has been initiated. Phagocytes have destroyed bacteria and debris, and the surface 9' of the injured tissue 9 is no more infected. When the piece 1 of the sponge-like material is removed from the injured tissue 9, a clean surface 8 of granulated tissue 10 is revealed. It can be observed that the granulated tissue 10' has also partially grown inside the piece 1 of sponge like material.

Figure 1c shows situation after skin transplation. A split skin autograft 11 has been placed on the clean surface 8 of the granulated tissue 10. An ultra thin autograft can be used as well. Some phagocytic cells 12 can be seen in granulated tissue 10. Blood vessels 3, 3' have grown to up to the autograft 11.

One method for preparation of the spongy cellulosic material comprising trace elements is described in example 1. Example 2 gives an exemplary use of the spongy cellulosic material comprising trace elements according to invention in the treatment of burns. Example 3 demonstrates demonstrates a rapid and quantitative binding of transforming growth factor beta 1 (TGF-β1) into cellulose sponges in vitro. All examples should be considered to be non-limiting.

### EXAMPLE 1

### Preparation of a spongy cellulosic material according to the invention

Cellulose viscose solution containing 5% of cellulose was prepared according to known methods. Calculated on the weight of the cellulose, 20% cotton fibres cut to the length of 8-10 mm, were mixed into the viscose solution. A pore forming material, in this case Glauber's salt (Na₂SO₄*10 H₂O) with a particle size < 1.0 mm was used. Glauber's salt was added 80 times the initial weight of the cellulose. The material obtained was placed into a mould and the cellulose was regenerated by heating in a water bath. The crude spongy cellulosic material leaving the water bath was washed salt free by means of hot water, treated with diluted acid, bleaching solution and finally washed repetitively with distilled water. The clean spongy cellulosic material was then washed repetitively with a solution, prepared in distilled water and containing such amounts of the desired trace elements that target concentrations of trace elements of the dried material were obtained. Trace elements are easily dissolved into a solution if corresponding chloride compounds are used. Amount of deposited trace elements was calculated by comparing the trace element concentrations in the washing solution before and after washing of the spongy cellulosic material. When the target concentrations deposited on the material were reached, the material was dried, cut to the desired size and packed.

### EXAMPLE 2

### Treatment of deep burns using material according to the invention.

Burned area of the skin was revised by removing the necrotised and devitalised parts of the tissue. This debridement was done carefully slice-by-slice, so deep that minimal bleeding was observed on the tissue. This indicated the boundary of the living tissue. One (1) litre of noncompressed spongy cellulosic material comprising trace elements was carefully moistened by imbibing with maximum of 580 - 600 ml of physiological saline solution (0.9 % NaCl). The material was cut to the form and size of the burned area. The moistened material according to invention was placed on the debrided raw surface of the burned tissue and covered with cellophane film. If the tissue was not infected, the material is changed once after three days. If the tissue is infected, in the beginning of the treatment the material is changed every day. The wound base is examined visually during the change and as the infection diminishes the change intervals are prolonged. The total treatment time for sufficient wound base granulation is normally 5-10 days and amount of changes varies between 2 and 5. The revascularisation of the burned area will usually occur within five days from the start of the treatment.

### EXAMPLE 3

### Demonstration of rapid and quantitative binding of transforming growth factor beta 1 (TGF-β1) into cellulose sponges in vitro

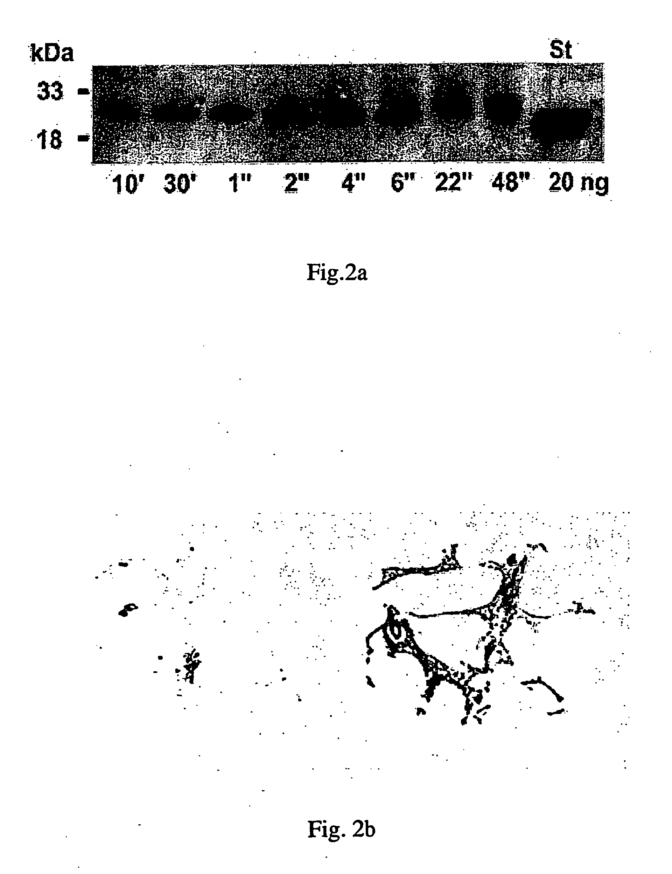

The rapid and quantitative binding of transforming growth factor beta 1 (TGF-β1) into sponge-like cellulosic material according to the present invention in vitro was analysed with Western analysis. The cellulosic material were cut into test pieces of size 5×5×5 mm. The test pieces were then incubated with 20 ng of purified TGF- β1 in phosphate buffered saline solution (PBS). Solution volume was five times the test piece volume. Incubation time was in different test 10 and 30 minutes, and 1, 2, 4, 6, 22 and 48 hours. After incubation the test pieces were washed with saline solution, and the TGF-β1 bound to the pieces was extracted into an electrophoresis buffer. The extracted TGF-β1 was run in polyacrylamide gel and transferred to Hybond ECL nitrocellulose membrane (Amersham Life Sciences, UK). Specific binding of TGF-β1 antibody (1.5 µg/ml, RDI, UK) was detected with Vectastain ABC kit (Vector Laboratories, USA) and visualised by enhanced chemiluminescence (ECL) detection system (Amersham, UK). Figure 2a shows the rapid and constant binding of TGF-β1 to the sponge-like cellulosic material according the present invention.

The rapid and quantitative binding of transforming growth factor beta 1 (TGF-β1) into sponge-like cellulosic material according to the present invention in vitro was also analysed with immunohistological analysis. Similar sponges were incubated with or without TGF beta as before. After rinsing the section with tris-buffered saline (TBS), the bound primary antibodies were visualized with the avidin-biotin-peroxidase complex technique (Vectastain ABC kit, Vector Laboratories, Inc., USA) using diaminobenzidine as chromogen. The tissues were counterstained with Mayer's hematoxylin stain. On the left in Figure 2b is shown the control sample and on the right the staining of TGF-β1 treated sponge. Figure 2b visualises the strong binding of the TGF-β1 to the structure of sponge-like cellulosic material according to the present invention.

It will be appreciated that the essence of the present invention can be corporated in the form of a variety of embodiments, only a few of which are disclosed herein. It will be apparent for the specialist in the field that other embodiments exist and do not depart from the spirit of the invention. Thus, the described embodiments are illustrative and should not be construed as restrictive.

## Claims

1. Porous sponge-like cellulosic material to be used in treatment of injuries and/or burns wherein the cellulosic material comprises zinc, copper, selenium and/or iron bound to and/or absorbed into the material matrix in amounts sufficient to support wound base granulation and revascularisation, the amount of zinc, copper, selenium and iron being at least 0.1 µg/g dry material.

2. Porous sponge-like cellulosic material according to claim 1, **characterised in that** the cellulosic material comprises in addition other trace elements.

3. Porous sponge-like cellulosic material according to claim 1, **characterised in that** the iron concentration is 5 - 28 µg/g dry material, preferably 10 - 14 µg/g dry material.

4. Porous sponge-like cellulosic material according to claim 1, **characterised in that** the zinc concentration is 2 - 20 µg/g dry material, preferably 7 - 10 µg/g dry material.

5. Porous sponge-like cellulosic material according to claim 1, **characterised in that** the copper concentration is 4 - 24 µg/g dry material, preferably 6 - 10 µg/g dry material.

6. Porous sponge-like cellulosic material according to claim 1, **characterised in that** the selenium concentration is 0.5 - 3.0 µg/g dry material, preferably 0.8 - 1.2 µg/g dry material.

7. Porous sponge-like cellulosic material according claim 1, **characterised in that** a semi-permeable film, through which water vapour can permeate but which detains proteins with molecular weight > 5000 in the material, is attached on one side of the cellulosic material.

8. The use of porous sponge-like cellulosic material according to any of the claims 1 to 6 for the manufacture of a device for treatment of acute wounds.

9. The use of porous sponge-like cellulosic material according to any of the claims 1 to 6 for the manufacture of a device for treatment of chronic ulcers.

10. The use of porous sponge-like cellulosic material according to any of the claims 1 to 6 for the manufacture of a device for treatment of chronic infections.

11. The use of porous sponge-like cellulosic material according to any of the claims 1 to 6 for the manufacture of a device for treatment of burns.

## Patentansprüche

1. Poröses, schwammartiges, zur Behandlung von Verletzungen und/oder Verbrennungen zu verwendendes Cellulosematerial, wobei das Cellulosematerial Zink, Kupfer, Selen und/oder Eisen, das an die Materialmatrix gebunden und/oder in die Materialmatrix absorbiert ist, in Mengen umfasst, die dazu ausreichend sind, eine Wundbasisgranulierung und -revaekularisierung zu fördern, wobei die Menge an Zink, Kupfer, Selen und Eisen mindestens 0,1 µg/g Trockenmaterial beträgt.

2. Poröses, schwammartiges Cellulosematerial nach Anspruch 1, **dadurch gekennzeichnet, dass** das Cellulosematerial zusätzlich andere Spurenelemente umfasst.

3. Poröses, schwammartiges Cellulosematerial nach Anspruch 1, **dadurch gekennzeichnet, dass** die Eisenkonzentration 5-28 µg/g Trockenmaterial, vorzugsweise 10-14 µg/g Trockenmaterial beträgt.

4. Poröses, schwammartiges Cellulosematerial nach Anspruch 1, **dadurch gekennzeichnet, dass** die Zinkkonzentration 2-20 µg/g Trockenmaterial, vorzugsweise 7-10 µg/g Trockenmaterial beträgt.

5. Poröses, schwammartiges Cellulosematerial nach Anspruch 1, **dadurch gekennzeichnet, dass** die Kupferkonzentration 4-24 µg/g Trockenmaterial, vorzugsweise 6-10 µg/g Trockenmaterial beträgt.

6. Poröses, schwammartiges Cellulosematerial nach Anspruch 1, **dadurch gekennzeichnet, dass** die Selenkonzentration 0,5-3,0 µg/g Trockenmaterial, vorzugsweise 0,8-1,2 µg/g Trockenmaterial beträgt.

7. Poröses, schwammartiges Cellulosematerial nach Anspruch 1, **dadurch gekennzeichnet, dass** ein semipermeabler Film, durch den Wasserdampf hindurchtreten kann, der jedoch Proteine mit einem Molekulargewicht > 5000 in dem Material zurückhält, an einer Seite des Cellulosematerials befestigt ist.

8. Verwendung des porösen, schwammartigen Cellulosematerials nach einem der Ansprüche 1-6 zur Herstellung einer Vorrichtung zur Behandlung akuter Wunden.

9. Verwendung des porösen, schwammartigen Cellulosematerials nach einem der Ansprüche 1-6 zur Herstellung einer Vorrichtung zur Behandlung chronischer Geschwüre.

10. Verwendung des porösen, schwammartigen Cellulosematerials nach einem der Ansprüche 1-6 zur Herstellung einer Vorrichtung zur Behandlung chronischer Infektionen.

11. Verwendung des porösen, schwammartigen Cellulosematerials nach einem der Ansprüche 1-6 zur Herstellung einer Vorrichtung zur Behandlung von Verbrennungen.

## Revendications

1. Matière cellulosique poreuse analogue à une éponge à utiliser dans le traitement de blessures et/ou de brûlures, la matière cellulosique comprenant du zinc, du cuivre, du sélénium et/ou du fer lié à et/ou absorbé dans la matrice de la matière, en quantités suffisantes pour supporter une granulation et une revascularisation à la base d'une plaie, la quantité de zinc, de cuivre, de sélénium et de fer étant d'au moins 0,1 µg/g de matière sèche.

2. Matière cellulosique poreuse analogue à une éponge selon la revendication 1, **caractérisée en ce que** la matière cellulosique comprend en outre d'autres éléments-trace.

3. Matière cellulosique poreuse analogue à une éponge selon la revendication 1, **caractérisée en ce que** la concentration en fer est de 5 à 28 µg/g de matière sèche, de préférence de 10 à 14 µg/g de matière sèche.

4. Matière cellulosique poreuse analogue à une éponge selon la revendication 1, **caractérisée en ce que** la concentration en zinc est de 2 à 20 µg/g de matière sèche, de préférence de 7 à 10 µg/g de matière sèche.

5. Matière cellulosique poreuse analogue à une éponge selon la revendication 1, **caractérisée en ce que** la concentration en cuivre est de 4 à 24 µg/g de matière sèche, de préférence de 6 à 10 µg/g de matière sèche.

6. Matière cellulosique poreuse analogue à une éponge selon la revendication 1, **caractérisée en ce que** la concentration en sélénium est de 0,5 à 3,0 µg/g de matière sèche, de préférence de 0,8 à 1,2 µg/g de matière sèche.

7. Matière cellulosique poreuse analogue à une éponge selon la revendication 1, **caractérisée en ce qu'**un film semi-perméable, à travers lequel la vapeur d'eau peut s'infiltrer mais qui retient les protéines de masse moléculaire supérieure à 5 000 dans la matière, est fixé sur un côté de la matière cellulosique.

8. Utilisation de matière cellulosique poreuse analogue à une éponge selon l'une quelconque des revendications 1 à 6 pour la fabrication d'un dispositif destiné au traitement de blessures aiguës.

9. Utilisation de matière cellulosique poreuse analogue à une éponge selon l'une quelconque des revendications 1 à 6 pour la fabrication d'un dispositif destiné au traitement d'ulcères chroniques.

10. Utilisation de matière cellulosique poreuse analogue à une éponge selon l'une quelconque des revendications 1 à 6 pour la fabrication d'un dispositif destiné au traitement d'infections chroniques.

11. utilisation de matière cellulosique poreuse analogue à une éponge selon l'une quelconque des revendications 1 à 6 pour la fabrication d'un dispositif destiné au traitement de brûlures.
